# EUROPEAN PATENT APPLICATION

(11) **EP 4 295 795 A1**
(43) Date of publication of application: **27.12.2023**
(21) Application number: 22180949.4
(22) Date of filing: 24.06.2022
(51) Int. Cl.: A61B 18/12, A61B 18/14, B26B 21/48, A61B 18/00

(54) **A HAIR CUTTING DEVICE AND AN ATTACHMENT THEREFORE**

(71) Applicant: Koninklijke Philips N.V., 5656 AG Eindhoven (NL)
(72) Inventor: PALERO, Jonathan Alambra, 5656 AG Eindhoven (NL); VARGHESE, Babu, 5656 AG Eindhoven (NL); DOODEMAN, Gerardus Johannes Nicolaas, 5656AG Eindhoven (NL); FRACKOWIAK, Bruno Jean François, 5656AG Eindhoven (NL); VISSER, Cornelis Gerardus, 5656 AG Eindhoven (NL); BOURQUIN, Yannyk Parulian Julian, 5656 AG Eindhoven (NL)
(74) Representative: Philips Intellectual Property & Standards

(57) **Abstract**

According to an aspect, there is provided an electric hair cutting device (100) comprising a body (102) comprising a radiofrequency, RF, generator unit (108) configured to generate RF energy; and a first plurality of electrodes (110) electrically coupled to the RF generator unit; and an attachment assembly (104) removably mountable to the body, the attachment assembly comprising a second plurality of electrodes (114) configured to contact the skin of a user during use; wherein the first plurality of electrodes are configured to be electrically coupled to the second plurality of electrodes of the attachment assembly when the attachment assembly is mounted to the body, such that RF energy generated by the RF generator unit is able to be transmitted to the skin of the user in order to increase the temperature of the skin of the user.

## Description

### FIELD OF THE INVENTION

The invention relates to an electric hair cutting device and an attachment for use with an electric hair cutting device and, more particularly, to an electric hair cutting device and an attachment which are capable of increasing a temperature of a user's skin.

### BACKGROUND OF THE INVENTION

A hair cutting device, such as an electric shaver, a hair trimmer or clippers, may be used to cur or shave hair on part of a person's body, such as their head or face. An improved hair cutting experience can be achieved for the user of such a device by warming up the part of person's body where the hair is to be cut.

One existing shaving device includes a component that heats up during use, and which transfers heat to the skin of the user. A downside of such direct heating methods is that the user can experience "hot spots" where the surface of the user's skin feels particularly hot in the region in contact with the shaving device, but little effect is experienced elsewhere. Furthermore, the heat from such direct heating is focussed at the skin surface, with little warming effect below the surface of the skin.

A further problem to be overcome is to provide components capable of achieving the warming effect in a handheld device, where space is limited.

There is therefore a desire for an alternative form of heating the skin of a user of a hair cutting device, which addresses the above-identified downsides.

### SUMMARY OF THE INVENTION

There is a need for a hair cutting device that can provide warmth to a user during use safely, in order to provide an improved user experience. The inventors of the present disclosure have recognised that such a warming experience can be achieved by generating radiofrequency (RF) energy using an RF generator unit, and transmitting the RF energy via electrodes into the user's skin. The electrodes may be located on the hair cutting device itself, or on an attachment (e.g., a comb attachment) that can be mounted onto the hair cutting device

According to a first specific aspect, there is provided an electric hair cutting device comprising a body comprising a radiofrequency, RF, generator unit configured to generate RF energy; and a first plurality of electrodes electrically coupled to the RF generator unit; and an attachment assembly removably mountable to the body, the attachment assembly comprising: a second plurality of electrodes configured to contact the skin of a user during use; wherein the first plurality of electrodes are configured to be electrically coupled to the second plurality of electrodes of the attachment assembly when the attachment assembly is mounted to the body, such that RF energy generated by the RF generator unit is able to be transmitted to the skin of the user in order to increase the temperature of the skin of the user.

In some embodiments, the RF generator unit may be configured to generate RF energy to be transmitted to the skin of the user such that a temperature of the skin of the user is caused to increase to a temperature of between 38°C and 42°C.

The RF generator unit may be configured to operate according to at least one of the following parameters:
i) an RF frequency of between 0.5 MHz and 100 MHz;
ii) an RF peak-to-peak voltage of between 10 Vpp and 100 Vpp; and
iii) a power rating of 20 W.

The electric hair cutting device may, in some embodiments, further comprise a processing unit in operative communication with the RF generator unit, the processing unit configured to control an operating parameter of the RF generating unit; and control an operating parameter of a cutting element of the electric hair cutting device.

In some embodiments, the electric hair cutting device may further comprise a skin impedance measurement unit configured to measure an impedance of the skin between a pair of electrodes in the second plurality of electrodes during use. The processing unit is configured to control an operating parameter of the RF generator unit based on the measured impedance of the skin.

The second plurality of electrodes may comprise a first pair of electrodes of opposite polarity and a second pair of electrodes of opposite polarity. The skin impedance measurement unit may be configured to measure an impedance of the skin between each electrode in the first pair of electrodes and between each electrode in the second pair of electrodes. The processing unit may be configured to control a parameter of the RF energy delivered to each of the first and second pairs of electrodes based on the measured impedances of the skin.

In some embodiments, the relative positions of the electrodes of the first plurality of electrodes and/or the second plurality of electrodes are adjustable.

The electric hair cutting device may further comprise at least one electrically insulating element located between adjacent electrodes of opposite polarity of the second plurality of electrodes, the at least one electrically insulating element configured to contact the skin of the user during use in order to restrict the flow of current between the adjacent electrodes of opposite polarity via fluid present on the skin of the user.

The second plurality of electrodes may comprise a first electrode of a first polarity, a second electrode of a second polarity and a third electrode of the second polarity. The second electrode and the third electrode may be located either side of the first electrode.

In some embodiments, a skin-contacting surface area of the first electrode may be between two and four times greater than the skin-contacting surface area of each of the second electrode and the third electrode.

The first electrode may have a width of between 2.8 mm and 7 mm, the second electrode may have a width of between 1 mm and 3.5 mm, and/or the third electrode may have a width of between 1 mm and 3.5 mm.

In some embodiments, a separation between the first electrode and each of the second and third electrodes may be between 3 mm and 15 mm.

According to a second specific aspect, there is provided an attachment for an electric hair cutting device, the attachment configured to be removably mounted to a body of the electric hair cutting device, the attachment comprising a plurality of electrodes configured to contact the skin of a user during use; and a contact element electrically coupled to the plurality of electrodes, and configured such that, when the attachment is mounted onto the body of the electric hair cutting device, the contact element is to receive radio frequency, RF, energy from an RF generator unit of the electric hair cutting device and to transmit the RF energy via the plurality of electrodes into the skin of the user in order to increase the temperature of the skin of the user.

According to a third specific aspect, there is provided an electric hair cutting device comprising a body comprising a radiofrequency, RF, generator unit configured to generate RF energy; and a cutting assembly comprising a cutting element; and a first plurality of electrodes electrically coupled to the RF generator unit and are configured such that, when the first plurality of electrodes contact the skin of a user RF energy is conducted from the RF generator unit into the skin of the user in order to increase the temperature of the skin of the user.

The electric hair cutting device may further comprise an attachment assembly removably mountable to the body, the attachment assembly comprising a second plurality of electrodes configured to contact the skin of a user during use. The first plurality of electrodes may be electrically coupled to the second plurality of electrodes of the attachment assembly such that, in use, RF energy can be conducted from the RF generator unit to the second plurality of electrodes and into the skin of the user in order to increase the temperature of the skin of the user.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of an example of an electric hair cutting device;
Fig. 2 is a schematic illustration of an example of an arrangement of electrodes;
Fig. 3 is a schematic illustration of a further example of an arrangement of electrodes;
Fig. 4 is a schematic illustration of a further example of an arrangement of electrodes;
Fig. 5 is a schematic illustration of a further example of an arrangement of electrodes;
Fig. 6 is a schematic illustration of a further example of an arrangement of electrodes;
Fig. 7 is a schematic illustration of an example of electrodes whose relative positions can be changed;
Fig. 8 is a schematic illustration of a further example of electrodes whose relative positions can be changed;
Fig. 9 is a schematic illustration of an example of an arrangement of electrodes with electrically insulating elements;
Fig. 10 is a schematic illustration of a further example of an arrangement of electrodes with electrically insulating elements;
Fig. 11 is a schematic illustration of a further example of an electric hair cutting device; and
Fig. 12 is an illustration of an example of an attachment for an electric hair cutting device.

### DETAILED DESCRIPTION OF THE EMBODIMENTS

According to embodiments disclosed herein, an electric hair cutting device is provided which can provide a warming effect in a user's skin, while the device is being used to cut or trim hair. The warming effect is achieved through the transmission of radiofrequency (RF) energy via a plurality of electrodes into the user's skin. In this way, the hair cutting experience is enhanced for the user. Furthermore, by warming the skin, the cutting effect of the hair cutting activity may be improved, for example, if a close shave is to be achieved.

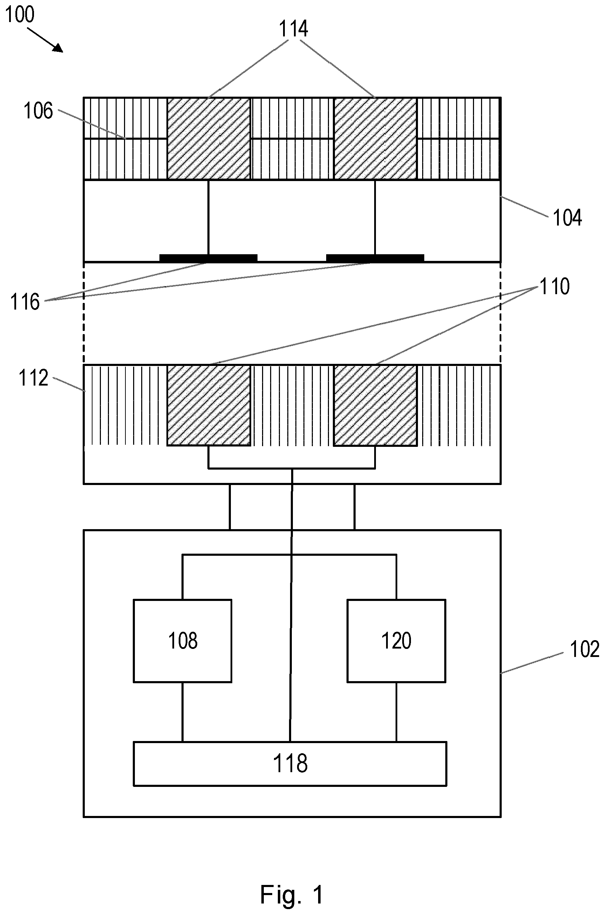

According to a first aspect, an electric hair cutting device is provided. Referring to the drawings, Fig. 1 is a schematic illustration of an example of an electric hair cutting device 100. The hair cutting device 100 may comprise a shaving device (e.g., an electric shaver), a hair trimming device (e.g., hair clippers) or any other electric device used for cutting hair. The hair cutting device 100 may, for example, comprise a handheld device, and may be powered by mains power, by one or more batteries and/or by one or more other power sources.

The electric hair cutting device 100 comprises a body 102 (also referred to as a body portion or housing) and an attachment assembly 104 which can be removably mounted to the body 102. For example, the attachment assembly 104 may include a coupling element or fixing to enable it to be mounted onto and removed from the body 102. The attachment assembly 104 may, in some embodiments, comprise an attachment that is configured to engage a user's skin during use, so as to separate the user's skin from a cutting element (e.g., blades) of the electric hair cutting device 100. For example, the attachment assembly 104 may comprise a comb attachment having teeth 106 that serve to separate and/or lift hairs to be cut during use.

The body 102 of the hair cutting device 100 comprises a radiofrequency (RF) generator unit 108 configured to generate RF energy. The body 102 also comprises a first plurality of electrodes 110 electrically coupled to the RF generator unit 108. For example, the first plurality of electrodes 110 may receive RF energy from the RF generator unit 108. In some embodiments, the first plurality of electrodes 110 may be located on or near to a cutting element 112 of the hair cutting device 100. The cutting element 112 may comprise one or more blades configured to cut hair is during use. The RF generator unit 108 may receive power from a power source (not shown) which, as noted above, may comprise a mains power supply and/or one or more batteries. The same power source may be used to provide power to other components in the electric hair cutting device 100.

The attachment assembly 104 comprises a second plurality of electrodes 114 configured to contact the skin of a user during use. For example, when the attachment assembly 104 is mounted onto the body 102 of the hair cutting device 100, the second plurality of electrodes 114 may engage the skin of the user during a hair cutting activity (e.g., when the hair cutting device is being used to cut the user's hair.

The first plurality of electrodes 110 are configured to be electrically coupled to the second plurality of electrodes 114 of the attachment assembly 104 when the attachment assembly is mounted to the body 102, such that RF energy generated by the RF generator unit 108 is able to be transmitted to the skin of the user in order to increase the temperature of the skin of the user. Each of the electrodes in the second plurality of electrodes 114 may, for example, include, or be electrically coupled to, one or more electrical contacts 116 located on the attachment assembly 104, which can engage a corresponding one or more electrodes of the first plurality of electrodes 110 on the body 102 of the hair cutting device 100. For example, when the attachment assembly 104 is mounted onto the body 102, an electrical connection may be formed between the first plurality of electrodes 110 and the second plurality of electrodes 114.

During use, RF energy generated by the RF generator unit 108 may pass via the first plurality of electrodes 110 and the second plurality of electrodes 114 into the user's skin (e.g., between a pair of electrodes of opposite polarity), thereby causing the temperature of the skin to increase, which results in a warming sensation for the user and also improves the effectiveness of the cutting activity. RF energy may be delivered to the user's skin while the electric hair cutting device 100 is being used to cut hair, or independently of the hair cutting function, for example when the cutting element 112 is not being operated.

The amount of RF energy generated by the RF generator unit 108, may be varied by varying one or more parameters of the RF generator unit. For example, by increasing the power supplied to the RF generator unit 108, the amount of RF energy generated may be increased, leading to a larger increase in temperature in the user's skin.

In some embodiments, the RF generator unit 108 may be configured to generate RF energy to be transmitted to the skin of the user such that a temperature of the skin of the user is caused to increase to a temperature of between 38°C and 42°C. In some examples, RF energy may be supplied such that the temperature of the skin of the user, at its surface, is caused to increase the temperature to a maximum of around 42°C, as this temperature can provide a pleasant warming feeling and improve the effectiveness of the hair cutting activity, without causing pain or discomfort to the user. With the temperature at the surface of the skin at around 42°C, the temperature within the epidermis and the dermis of the skin may reach between around 38°C and 40°C. Such a temperature increase deep in the tissue of the user can provide a pleasant warming sensation.

The desired temperature increase in the user's skin may be achieved by adjusting one or more parameters of the electric hair cutting device 100 to vary the amount of RF energy that is transmitted into the user's skin. For example, one or more parameters of the RF generator unit 108 may be varied. The RF generator unit 108 may be configured to configured to operate according to one or more particular parameters, such as those discussed below. For example, the RF generator unit 108 may be configured to operate with an RF frequency of between 0.5 MHz and 100 MHz, or more preferably between 0.5 MHz and 50 MHz, or even more preferably between 0.5 MHz and 10 MHz. In some examples, the RF generator unit 108 may be configured to operate with an RF peak-to-peak voltage of between 10 Vpp and 100 Vpp. In some examples, the RF generator unit 108 may be configured to operate with a power rating of 20 W. For a typical user, with typical skin properties, electrode geometries and RF parameters (e.g., frequency and voltage), an RF generator unit 108 configured in this way will deliver RF energy to the user's skin with an RF power dissipation of between 1 W and 20 W. The RF power dissipation may be varied (e.g., by adjusting a parameter of the RF generator unit 108) depending on the electrode geometry (e.g., the size and arrangement of the first plurality of electrodes and/or the second plurality of electrodes) and/or on an RF voltage input (i.e., the voltage delivered to the user's skin supplied by the RF generator unit 108). In other words, the RF generator unit 108 may be configured to deliver RF energy to the user's skin with an RF power dissipation of between 1 W and 20 W. One or more of the above parameters may be adjusted, along with one or more other parameters of the RF generator unit 108, in order to generate RF energy suitable for increasing the temperature of the user's skin to within the intended temperature range.

The parameters of the RF generator unit 108 may be adjusted and/or controlled by one or more processors or processing units located within the electric hair cutting device 100 or located remotely with respect to the electric hair cutting device (e.g., in wireless communication with the RF generator unit 108). In some examples, the electric hair cutting device 100 may further comprise a processing unit 118 in operative communication with the RF generator unit 108. The processing unit 118 may be configured to control an operating parameter of the RF generating unit 108. In some embodiments, the processing unit 118 may be further configured to control an operating parameter of the cutting element 112 of the hair cutting device 100. For example, the processing unit 118 may control a motor to cause one or more blades of the cutting element 112 to rotate or move, and/or the processing unit may adjust the relative positioning of blades in the cutting element to change a hair cutting length to be achieved by the hair cutting device 100. In other examples, the processing unit 118 may be configured to control other components of the electric hair cutting device 100.

In general, RF energy generated by the RF generator unit 108 is transmitted through the user's skin, from a first electrode to a second electrode. In examples in which the electric hair cutting device 100 is used without the attachment assembly 104, the RF energy may be transmitted from a first electrode of the first plurality of electrodes, through the skin, to a second electrode of the first plurality of electrodes and, in examples in which the attachment assembly is attached to the body 102 during use, then the RF energy may be transmitted from a first electrode of the second plurality of electrodes, through the skin, to a second electrode of the second plurality of electrodes.

In some embodiments, the amount of RF energy generated by the RF generator unit 108 may remain constant during use. In other embodiments, however, one or more parameters of the RF generator unit 108 may be adjusted during use to vary the amount of RF energy provided to the skin of the user, thereby varying the temperature change experienced by the user. In one example, a skin temperature measuring component may be provided to measure a temperature of the skin near to the location in contact with one or more of the electrodes. If it is determined that the temperature of the skin meets or exceeds a threshold temperature, then one or more parameters of the RF generator unit 108 may be adjusted to reduce the amount of RF energy generated. Thus, one or more parameters of the RF generator unit 108 may be adjusted based on the measured skin temperature.

According to some embodiments, the electric hair cutting device 100 may further comprise a skin impedance measurement unit 120 configured to measure an impedance of the skin between a pair of electrodes during use (e.g., between a pair of electrodes in the first plurality of electrodes if the hair cutting device is used without the attachment assembly 104, or between a pair of electrodes in the second plurality of electrodes if the attachment assembly is used). Skin impedance of a user's skin may vary depending on how wet the skin is, or how much fluid is present on the surface of the skin. For example, if the user applies a gel or fluid (shaving gel or water) to their skin prior to commencing a shaving activity, then the impedance of their skin may be different to the impedance of their skin if no gel or fluid is applied. The processing unit 118, which may be in operative communication with the skin impedance measurement unit 120, may be configured to control an operating parameter of the RF generator unit 108 based on the measured impedance of the skin. The power and/or peak-to-peak voltage of the RF generating unit 108 may be varied based on the measured skin impedance, for example. If, for example, it is determined that the impedance of the skin is below a threshold impedance, then the processing unit 118 may control the RF generator unit 108 to reduce the amount/power of RF energy generated.

In some embodiments, the first plurality of electrodes and/or the second plurality of electrodes may comprise multiple pairs of electrodes, such as multiple discrete pairs of electrodes or multiple pairs of electrodes that share a common electrode. In such embodiments, the skin impedance measurement unit 120 may be configured to measure an impedance of the skin between each pair of electrodes, and an operating parameter of the RF generator unit 108 may be adjusted to vary a parameter of the RF energy (e.g., the power and/or peak-to-peak voltage) supplied to each electrode or pair of electrodes based on the measured skin impedance at each electrode or pair of electrodes. In this way, the RF energy supplied to the user's skin can be adjusted taking into account the differing levels of wetness of the user's skin over the extent of the cutting element 112 or the attachment assembly 104 in contact with the user's skin during use. Thus, in some embodiments, the second plurality of electrodes 114 (or the first plurality of electrodes if the electric hair cutting device 100 is to be used without the attachment assembly 104) may comprise a first pair of electrodes of opposite polarity and a second pair of electrodes of opposite polarity. The skin impedance measurement unit 120 may be configured to measure an impedance of the skin between each electrode in the first pair of electrodes and between each electrode in the second pair of electrodes. The processing unit 118 may be configured to control a parameter of the RF energy delivered to each of the first and second pairs of electrodes (or control a parameter of the RF generating unit 108) based on the measured impedances of the skin.

In another embodiment, the frequency of the RF energy supplied to the user's skin via the electrodes may be varied from user to user. While there exists an optimal range of frequencies for achieving a desired target skin temperature of 38°C to 42°C, the optimal frequency may be different for different users. Thus, prior to using the electric hair cutting device 100, a user may perform a calibration or probing step such that an optimal frequency of the RF energy can be determined. During such a calibration, a user may position the electric hair cutting device 100 such that the first plurality of electrodes 110 or the second plurality of electrodes 114 are in contact with the skin where pairs are to be cut. The RF generator unit 108 may then generate RF energy having at least three different frequencies, and the generated RF energy may be provided through the user's skin, across each of a plurality of pairs of electrodes. The skin impedance measurement unit 120 may then measure the impedance of the skin across each pair of electrodes, when the RF energy at the multiple different frequencies is supplied. From a slope of a plot of the measured skin impedance at each frequency of RF energy, an optimum frequency for the user can be determined. Once the optimum frequency for a particular user has been determined, the processing unit 118 may control the RF generator unit 108 to generate RF energy at the optimum frequency. In some examples, the optimum frequency for a particular user may be stored in a memory, for example as part of the user profile, so that it can be used for the same user during future hair cutting activities.

In addition to varying parameters of the RF generator unit 108, different warming effects in the user's skin can be achieved with different numbers and arrangements (e.g., sizes and relative positions) of the electrodes. While a minimum of two electrodes may be provided to transmit RF energy into the user's skin and achieve the intended warming sensation, three or more electrodes may alternatively be provided. With more electrodes, the warming effect may be increased, but more space is used on the cutting element 112 or the attachment assembly 104. Therefore, a compromise is needed to achieve a good skin-warming effect while maintaining a good cutting ability. In a preferred embodiment, three electrodes may be provided.

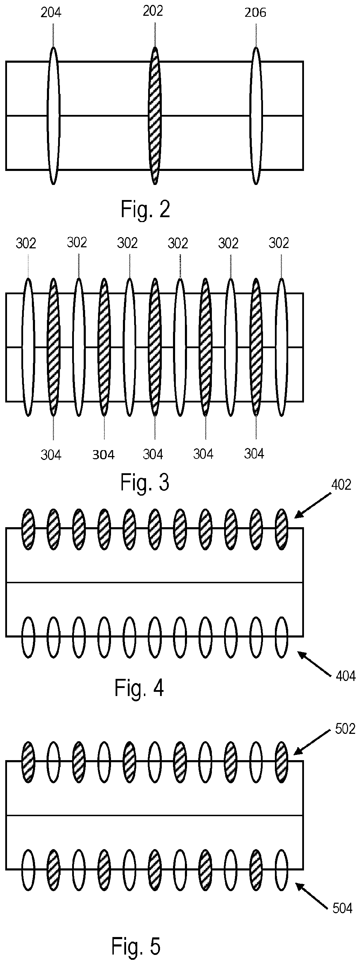

Figs. 2 to 6 show various possible arrangements of electrodes. Electrodes in the first plurality of electrodes 110 and/or the second plurality of electrodes 114 may be arranged according to the arrangements shown in Figs. 2 to 6.

In some embodiments, the first plurality of electrodes 110 and/or the second plurality of electrodes 114 may be arranged according to the arrangement shown in Fig. 2, in which the plurality of electrodes comprises a first electrode 202 of a first polarity, a second electrode 204 of a second polarity and third electrode 206 of the second polarity. In this example, the second electrode 204 and the third electrode 206 are located either side of the first electrode 202.

In the arrangement shown in Fig. 3, the plurality of electrodes alternate in polarity, such that adjacent electrodes are of opposite polarities (electrodes 302 are of a first polarity and electrodes 304 of a second polarity). In this example, the plurality of electrodes are arranged in a single row. In the arrangement shown in Fig. 4, the plurality of electrodes are arranged in two rows, a first row of electrodes 402 having a first polarity and a second row of electrodes 404 having a second, opposite polarity.

In the arrangement shown in Fig. 5, the plurality of electrodes are arranged in a first row 502 and a second row 504, and in each row, adjacent electrodes are of opposite polarities. The arrangement is such that electrodes in the first row 502 are opposite electrodes in the second row 504 that have opposing polarities.

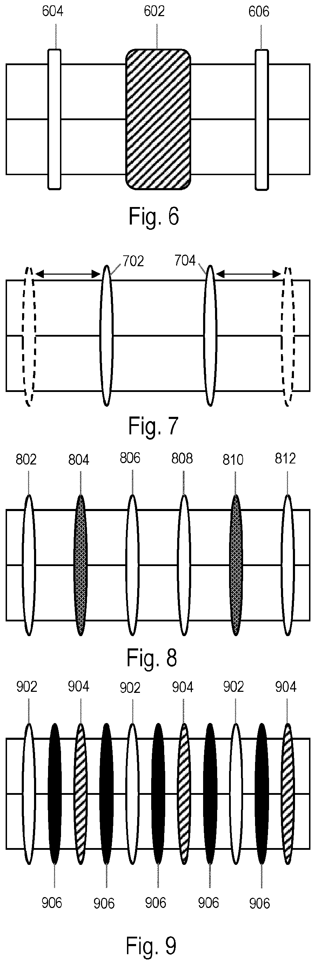

Fig. 6 shows an arrangement which is similar to the arrangement shown in Fig. 2, in which a first electrode 602 is arranged centrally with respect to a second electrode 604 and a third electrode 606. In the arrangement shown in Fig. 6, the first, central electrode 602 has a skin-contacting surface area that is larger than the second and third electrodes 604, 606 located either side. In some embodiments, a skin-contacting surface area of the first electrode 602 may be between two and four times greater than the skin-contacting surface area of each of the second electrode 604 and the third electrode 606. In a preferred embodiment, the skin-contacting surface area of the first electrode 602 may be between 2.5 and 3 times greater than the skin-contacting surface area of each of the second electrode 604 and the third electrode 606. In one embodiment, the skin-contacting surface area of the first electrode 602 may be 2.8 times greater than the skin-contacting surface area of each of the second electrode 604 and the third electrode 606. By arranging the electrodes with skin-contacting surface area ratio of between 2:1 and 4:1, a good balance of current density between the electrodes can be achieved. This leads to improved heating performance compared to other surface area ratios, and ensures that a good heating performance is maintained when smaller electrodes are used.

The size of each electrode can also affect the heating performance and/or a depth of tissue that is heated by the electrodes. In some embodiments, where the first plurality of electrodes 110 and/or the second plurality of electrodes 114 comprises three electrodes, arranged as shown in Fig. 6, the first, central electrode 602 may have a dimension (e.g., a width) that is greater than an equivalent dimension of the second and third electrodes 604, 606 located either side of the central electrode. For example, the length of all of the electrodes may be substantially the same, and this length may correspond to or be similar to a width of the cutting element 112 and/or a width of the attachment assembly 104. For example, the length of the electrodes may be around 20 mm. In some embodiments, the first electrode 602 may have a width of between 2.8 mm and 7 mm. While, in general, a better heating performance can be achieved with larger electrodes, the maximum size (e.g., width) of each electrode is constrained by the area available on the cutting element 112 and/or the attachment assembly 104. Thus, there is a trade-off between providing large electrodes for improved heating performance and maintaining adequate cutting capabilities by leaving enough of the cutting element 112 and/or the attachment assembly 104 exposed. In some embodiments, the second electrode 604 and/or the third electrode 606 may have a width of between 1 mm and 3.5 mm. In one particular example, the first electrode 602 may have a width of approximately 7 mm and the second electrode 604 and the third electrode 606 may have widths of approximately 2.5 mm. The electrodes may be any shape, such as rectangular or cuboidal. In some examples, the skin-contacting surface of an electrode may be flat (i.e., planar), while in other examples, the skin-contacting surface may be curved. The electrodes may be made of any electrically conductive material capable of conducting RF energy into the skin of a user. For example, the electrodes may be formed from metal.

The spacing between the electrodes may also affect the heating performance and/or a depth of tissue that is heated by the electrodes. In general, the smaller the gap between adjacent electrodes, the better the heating performance, due to a better steering of the RF current between the electrodes. However, if the separation between adjacent electrodes is too small, then this could result in current concentration, leading to hot spots, which could cause pain to the user. As with the selection of the sizes of the electrodes, a larger gap between adjacent electrodes may provide improved hair cutting functionality. In some examples, a separation between the first electrode 602 and each of the second and third electrodes 604, 606 may be between 3 mm and 15 mm. More preferably, in some embodiments, the separation may be between 3.4 m and 14.3 mm. In another particular embodiment, the separation may be between 3 mm and 4 mm.

In some embodiments, the optimal separation between adjacent electrodes may depend upon the widths of the electrodes. Thus, in some examples, the relative positions of the electrodes of the first plurality of electrodes and/or the second plurality of electrodes are adjustable. Figs. 7 and 8 are schematic illustrations of examples of how the relative positions of the plurality of electrodes may be adjusted.

In Fig. 7, a pair of electrodes 702, 704 is shown, though it will be appreciated that the relative positions of electrodes may be adjusted when any number of electrodes are provided. In the example shown in Fig. 7, one or more of the electrodes 702, 704 may be attached to all mounted on a movement mechanism, such that at least one of the electrodes can be moved (e.g., slid along a rail) relative to another electrode, so as to adjust the relative positions of the electrodes and to adjust the separation between adjacent electrodes.

In the example shown in Fig. 8, six electrodes 802 to 812 are shown, though again it will be appreciated that the following discussion is applicable to any number of electrodes. In this example, the electrodes themselves are stationary and are not movable. However, a mechanism is provided which enables different ones of the electrodes 802 to 812 to be used to transmit RF energy into the user's skin at any particular time. For example, the mechanism may involve a plurality of switches to mechanically connect and/or disconnect particular electrodes to/from the RF generator unit 108 as required. In some embodiments, the processor 118 may be configured to control which of the electrodes 802 to 812 is active at any particular time. In this way, the effective separation between the electrodes can be controlled. In Fig. 8, shaded electrodes 804 and 810 are indicated as being active. For example, if the electrodes 806 and 808 are active, but the other electrodes are inactive, then the effective separation between the active electrodes is relatively small, while if the electrodes 802 and 812 are active, but the other electrodes are inactive, then the effective separation between the active electrodes is relatively large.

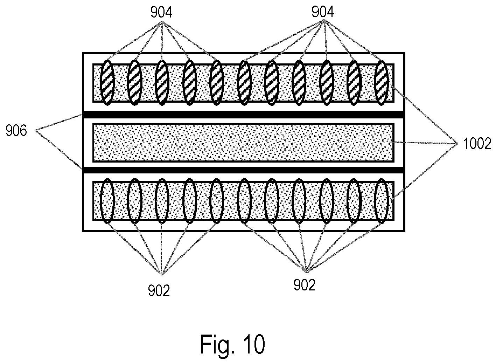

As discussed herein, the RF energy transmitted between the electrodes is intended to pass through the skin and tissue of the user. In some cases, however, fluid on the user's skin (e.g., water, sweat, moisturiser, shaving gel, or the like, may create an electrical connection between the electrodes, causing an effective "short circuit" situation, whereby the RF energy is transmitted between the electrodes through the fluid, rather than through the skin of the user. In order to reduce the likelihood that the RF energy is transmitted via the fluid, in some embodiments, an electrically insulating (i.e., electrically non-conductive) element may be provided to prevent such a short circuit event. The electrically insulating element may be formed of any electrically non-conductive material, such as a plastics material. Figs. 9 and 10 are schematic illustrations of examples of how such insulating element may be implemented.

In Fig. 9, a plurality of electrodes (e.g., the first or second plurality of electrodes 110, 114) are shown. The plurality of electrodes includes a first subset of electrodes 902 having a first polarity and a second subset of electrodes 904 having a second polarity. An electrically insulating element 906 is located between each pair of adjacent electrodes 902, 904. In use, the electrically insulating elements 906 engage the skin of the user, and restrict the flow of current between adjacent electrodes of opposite polarity 902, 904, unless the RF energy passes through the skin of the user.

A different arrangement of the plurality of electrodes 902, 904 is shown in Fig. 10. In this example, the electrodes 902, 904 are arranged such that electrodes of a first polarity 902 are arranged in a first row and electrodes of a second polarity 904 are arranged in a second row. In this example, multiple cutting elements 1002 (e.g., rotating blades) are provided and an electrically insulating element 906 is provided between each cutting element 1002, to restrict the flow of RF energy directly from the electrodes of the first polarity 902 to the electrodes of the second polarity 904, unless the RF energy travels via the user's skin.

Thus, more generally, the electric hair cutting device 100 may comprise at least one electrically insulating element 906 located between adjacent electrodes 902, 904 of opposite polarity of the second plurality of electrodes 114 (and/or of the first plurality of electrodes 110). The at least one electrically insulating element 906 is configured to contact the skin of the user during use in order to restrict the flow of current between the adjacent electrodes of opposite polarity via fluid present on the skin of the user.

Without an electrically insulating element between adjacent electrodes, any fluid around the electrodes acts as a conductive material, thus directing current from the sides of an electrode towards the skin surface. This increases the equivalent contact surface between the electrode and the skin, compared to the actual skin-contacting area of the electrode, thus steering more RF current towards the skin surface, rather than deeper into the skin, compared to when no fluid is present on the skin.

As discussed herein, the functionality concerning the electrodes of the present invention can be implemented into electrodes on the cutting element 112 (e.g., the first plurality of electrodes 110) and/or into electrodes on the attachment assembly 104 (e.g., the second plurality of electrodes 114). Thus, while the embodiment shown in Fig. 1 includes both the body 102 of the electric hair cutting device 100 and the attachment assembly 104, a further aspect of the invention relates to an electric hair cutting device that does not include the attachment assembly.

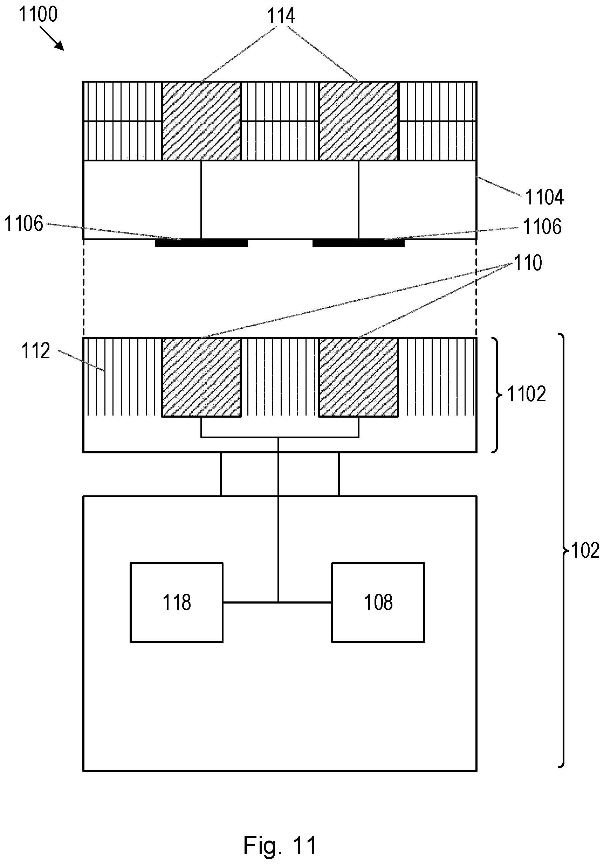

Fig. 11 is a schematic illustration of an example of an electric hair cutting device 1100 comprising a body 102. The body 102 comprises a radiofrequency (RF) generator unit 108 configured to generate RF energy, and a cutting assembly 1102 comprising a cutting element 112 and a first plurality of electrodes 110. The first plurality of electrodes 110 are electrically coupled to the RF generator unit 108 and are configured such that, when the first plurality of electrodes contact the skin of a user RF energy is conducted from the RF generator unit into the skin of the user in order to increase the temperature of the skin of the user.

Thus, the electric hair cutting device 1100 may provide the benefits of the disclosed invention without the use of an attachment assembly. Optionally, the attachment assembly 104 may be provided, for example to function as a comb, to lift and/or separate hair is being cut, while still providing the benefits of the invention. Thus, the electric hair cutting device 1100 may further comprise an attachment assembly 104 removably mountable to the body 102. The attachment assembly 104 may comprise a second plurality of electrodes 114 configured to contact the skin of a user during use. The first plurality of electrodes 110 may be electrically coupled to the second plurality of electrodes 114 of the attachment assembly 104 such that, in use, RF energy can be conducted from the RF generator unit 108 to the second plurality of electrodes and into the skin of the user in order to increase the temperature of the skin of the user. In some examples, the second plurality of electrodes 114 may be electrically coupled to at least one electrical contact 1106 configured to engage one or more of the first plurality of electrodes 110, when the attachment assembly 104 is mounted on the body 102.

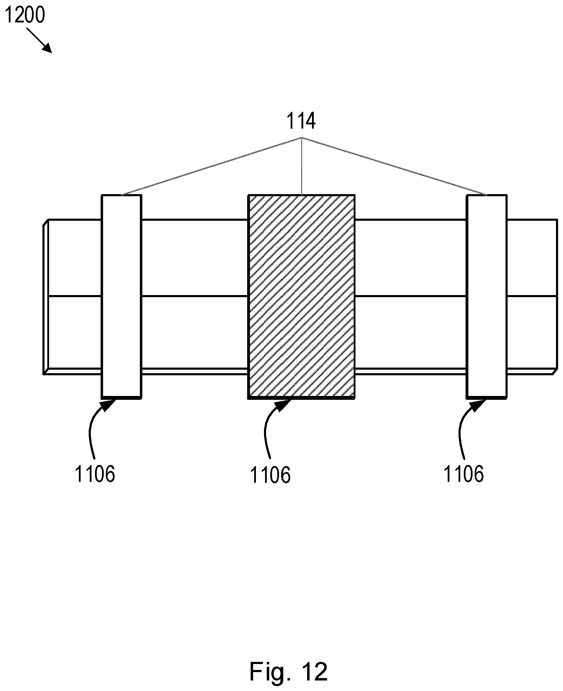

According to a further aspect, the present invention provides an attachment for an electric hair cutting device. Fig. 12 is an illustration of an example of an attachment 1200, which may comprise or be similar to the attachment assembly 104. The attachment 1200, which is suitable for use with an electric hair cutting device such as the device 1100, is configured to be removably mounted to a body 102 of the electric hair cutting device. The attachment 1200 comprises a plurality of electrodes 114 configured to contact the skin of a user during use. The attachment 1200 further comprises a contact element 1106 electrically coupled to the plurality of electrodes 114, and configured such that, when the attachment is mounted onto the body 102 of the electric hair cutting device 1100, the contact element is to receive radio frequency, RF, energy from an RF generator unit 108 of the electric hair cutting device and to transmit the RF energy via the plurality of electrodes into the skin of the user in order to increase the temperature of the skin of the user. In some examples, multiple contact element 1106 may be provided. The attachment 1200 may comprise any number of the features of the attachment assembly 104 disclosed herein.

Thus, the present disclosure provides a mechanism by which a warming effect can be provided to the skin of a user, when using an electric hair cutting device or an attachment thereof, to perform a hair cutting activity. Radiofrequency energy provides a pleasant warming experience in a person's skin, and the electric hair cutting devices and attachment assemblies according to the disclosed embodiments enable a user to experience such warming in their skin.

The processing unit 118 can comprise one or more processors, processing units, multicore processors or modules that are configured or programmed to control components of the electric hair cutting device and/or assembly in the manner described herein. In particular implementations, the processing unit 118 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g., Java interpreter instructions). Alternatively, one or more or all of the sub-routines may be stored in at least one external library file and linked with a main program either statically or dynamically, e.g., at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An electric hair cutting device (100) comprising:
a body (102) comprising:
a radiofrequency, RF, generator unit (108) configured to generate RF energy; and
a first plurality of electrodes (110) electrically coupled to the RF generator unit; and
an attachment assembly (104) removably mountable to the body, the attachment assembly comprising:
a second plurality of electrodes (114) configured to contact the skin of a user during use;
wherein the first plurality of electrodes are configured to be electrically coupled to the second plurality of electrodes of the attachment assembly when the attachment assembly is mounted to the body, such that RF energy generated by the RF generator unit is able to be transmitted to the skin of the user in order to increase the temperature of the skin of the user.

2. An electric hair cutting device (100) according to claim 1, wherein the RF generator unit (108) is configured to generate RF energy to be transmitted to the skin of the user such that a temperature of the skin of the user is caused to increase to a temperature of between 38°C and 42°C.

3. An electric hair cutting device (100) according to claim 1 or claim 2, wherein the RF generator unit (108) is configured to operate according to at least one of the following parameters:
i) an RF frequency of between 0.5 MHz and 100 MHz;
ii) an RF peak-to-peak voltage of between 10 Vpp and 100 Vpp; and
iii) a power rating of 20 W.

4. An electric hair cutting device (100) according to any of the preceding claims, further comprising:
a processing unit (118) in operative communication with the RF generator unit (108), the processing unit configured to:
control an operating parameter of the RF generating unit; and
control an operating parameter of a cutting element (112) of the electric hair cutting device.

5. An electric hair cutting device (100) according to claim 4, further comprising:
a skin impedance measurement unit (120) configured to measure an impedance of the skin between a pair of electrodes in the second plurality of electrodes (114) during use;
wherein the processing unit (118) is configured to:
control an operating parameter of the RF generator unit (108) based on the measured impedance of the skin.

6. An electric hair cutting device (100) according to claim 5, wherein the second plurality of electrodes (114) comprise a first pair of electrodes of opposite polarity and a second pair of electrodes of opposite polarity;
wherein the skin impedance measurement unit (120) is configured to measure an impedance of the skin between each electrode in the first pair of electrodes and between each electrode in the second pair of electrodes; and
wherein the processing unit (118) is configured to:
control a parameter of the RF energy delivered to each of the first and second pairs of electrodes based on the measured impedances of the skin.

7. An electric hair cutting device (100) according to any of the preceding claims, wherein the relative positions of the electrodes of the first plurality of electrodes and/or the second plurality of electrodes are adjustable.

8. An electric hair cutting device (100) according to any of the preceding claims, further comprising:
at least one electrically insulating element (906) located between adjacent electrodes of opposite polarity of the second plurality of electrodes, the at least one electrically insulating element configured to contact the skin of the user during use in order to restrict the flow of current between the adjacent electrodes of opposite polarity via fluid present on the skin of the user.

9. An electric hair cutting device (100) according to any of the preceding claims, wherein the second plurality of electrodes (114) comprise a first electrode of a first polarity, a second electrode of a second polarity and a third electrode of the second polarity;
wherein the second electrode and the third electrode are located either side of the first electrode.

10. An electric hair cutting device (100) according to claim 9, wherein a skin-contacting surface area of the first electrode is between two and four times greater than the skin-contacting surface area of each of the second electrode and the third electrode.

11. An electric hair cutting device (100) according to claim 9 or claim 10,
wherein the first electrode has a width of between 2.8 mm and 7 mm;
wherein the second electrode has a width of between 1 mm and 3.5 mm; and
wherein the third electrode has a width of between 1 mm and 3.5 mm.

12. An electric hair cutting device (100) according to any of claims 9 to 11, wherein a separation between the first electrode and each of the second and third electrodes is between 3 mm and 15 mm.

13. An attachment (1200) for an electric hair cutting device (100, 1100), the attachment configured to be removably mounted to a body of the electric hair cutting device, the attachment comprising:
a plurality of electrodes (114) configured to contact the skin of a user during use; and
a contact element (1106) electrically coupled to the plurality of electrodes, and configured such that, when the attachment is mounted onto the body of the electric hair cutting device, the contact element is to receive radio frequency, RF, energy from an RF generator unit of the electric hair cutting device and to transmit the RF energy via the plurality of electrodes into the skin of the user in order to increase the temperature of the skin of the user.

14. An electric hair cutting device (100, 1100) comprising:
a body (102) comprising:
a radiofrequency, RF, generator unit (108) configured to generate RF energy; and
a cutting assembly (1102) comprising:
a cutting element (112); and
a first plurality of electrodes (110) electrically coupled to the RF generator unit and are configured such that, when the first plurality of electrodes contact the skin of a user RF energy is conducted from the RF generator unit into the skin of the user in order to increase the temperature of the skin of the user.

15. An electric hair cutting device (100, 1100) according to claim 14, further comprising:
an attachment assembly (104) removably mountable to the body, the attachment assembly comprising:
a second plurality of electrodes (114) configured to contact the skin of a user during use;
wherein the first plurality of electrodes are electrically coupled to the second plurality of electrodes of the attachment assembly such that, in use, RF energy can be conducted from the RF generator unit to the second plurality of electrodes and into the skin of the user in order to increase the temperature of the skin of the user.
